# EUROPÄISCHE PATENTANMELDUNG

(11) **EP 0 558 444 A2**
(43) Veröffentlichungstag der Anmeldung: **01.09.1993**
(21) Anmeldenummer: 93810080.7
(22) Anmeldetag: 09.02.1993
(51) Int. Cl.: C07D 517/06, H01B 1/12

(54) **Verfahren zur Herstellung von Tetraselenotetracenhalogeniden und elektrisch leitenden Kunststoffzusammensetzungen**

(30) Priorität: 18.02.1992 CH 472/92
(71) Anmelder: CIBA-GEIGY AG, CH-4002 Basel (CH)
(72) Erfinder: Mayer, Carl W., Dr., CH-4125 Riehen (CH); Minder, Ernst, CH-4450 Sissach (CH)

(57) **Zusammenfassung**

Verfahren zur Herstellung von Tetraselenotetracenchloriden oder -bromiden der Formel I, 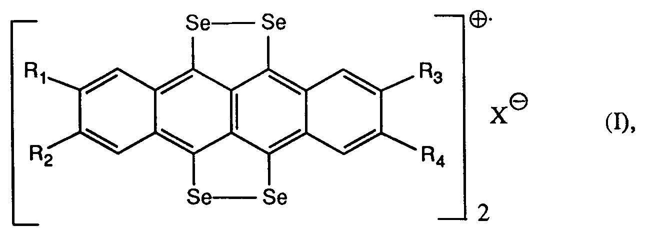
worin R₁, R₂, R₃ und R₄ unabhängig voneinander H oder F bedeuten; R₁ für CH₃ und R₂, R₃ und R₄ für H stehen; R₁, R₂, R₃ und R₄ je für CH₃ stehen; oder R₁ und R₂ CH₃ oder Cl und R₃ und R₄ H darstellen, und X für Cl oder Br steht, das dadurch gekennzeichnet ist, dass man ein Tetraselenotetracen der Formel II 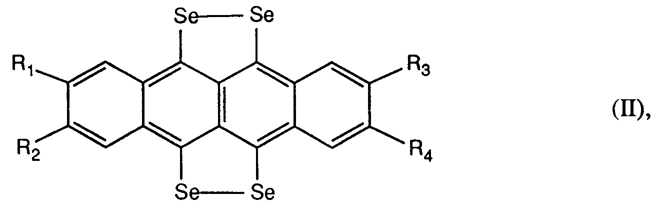
in einem polaren aprotischen Lösungsmittel mit stöchiometrischen Mengen eines Ammoniumhydrochlorids oder -bromids in Gegenwart von Sauerstoff bei erhöhter Temperatur umsetzt.
In der Reaktionslösung kann ein organisches Bindemittel gelöst sein und mit dieser Lösung können Substrate beschichtet werden, wobei man nach dem Verdampfen des Lösungsmittels elektrisch leitende Beschichtungen erhält, die aus einem Nadelnetzwerk von Kristallnadeln der Verbindungen der Formel I in der Bindemittelmatrix bestehen.

## Beschreibung

Die Erfindung betrifft ein Verfahren zur Herstellung von (Tetraselenotetracenen)₂Cl oder (Tetraselenotetracenen)₂Br durch die Umsetzung von Tetraselenotetracen mit Sauerstoff und einem Ammoniumhydrochlorid oder -bromid in Gegenwart eines polaren und aprotischen Lösungsmittes bei erhöhten Temperaturen. Die Lösung kann ein organisches Bindemittel enthalten, wobei nach dem Abdampfen des Lösungsmittels und gegebenenfalls überschüssiger Reaktanden und flüchtiger Reaktionsprodukte Zusammensetzungen zurückbleiben, die ein (Tetraselenotetracen)₂Cl oder (Tetraselenotetracen)₂Br in Form eines Netzwerks von Kristallnadeln in der Bindemittelmatrix enthalten.

J. Finter et al. beschreiben in Synthetic Metals, 41-43, Seiten 951 bis 954 die Herstellung von elektrisch leitenden Filmen aus (Tetraselenotetracen)₂Cl in Form eines Nadelnetzwerks von Kristallnadeln in einer Polymermatrix durch eine in situ Kristallisation bei der Oxidation von Tetraselenotetracen mit zum Beispiel Hexachloropropen in einem Lösungsmittel, die den Kunststoff enthält, bei erhöhter Temperatur unter Abdampfen des Lösungsmittels und gegebenenfalls anderer flüchtiger Bestandteile. Bei der Reaktion enstehen Substanzen, die Metallteile von Verarbeitungsmaschinen wie zum Beispiel Walzen oder Förderbänder korrodieren und daher aufwendige Schutzmassnahmen erfordern. Es besteht der Wunsch nach einem Oxidationsverfahren zur Herstellung solcher Filme, bei dem keine Korrosion von metallischen Maschinenteilen beobachtet wird und leitende Filme gleicher oder sogar besserer Qualität hergestellt werden können. Es besteht ferner der Wunsch nach einem Herstellverfahren von Tetraselenotetracenchloriden und -bromiden, bei dem keine Korrosion von Metallteilen der Produktionsanlage durch die Reaktionsmischung erfolgt.

Es wurde nun überraschend gefunden, dass die Oxidation von Tetraselenotetracenen in einem polaren und aprotischen Lösungsmittel mit einem Ammoniumhydrochlorid oder -bromid und Luftsauerstoff erfolgt, und hierbei nadelförmige Kristalle selbst in Gegenwart eines organischen Bindemittels entstehen. Es wurde ferner überraschend gefunden, dass die Reaktionsmischung trotz der Anwesenheit von Halogenid keine oder höchstens eine unwesentliche korrosive Wirkung auf Metallteile von Verarbeitungsmaschinen ausübt. Das Verfahren ist daher besonders für den technischen Einsatz und ein wirtschaftliches Produktionsverfahren geeignet.

Gegenstand der Erfindung ist somit ein Verfahren zur Herstellung von Tetraselenotetracenchloriden oder -bromiden der Formel I, 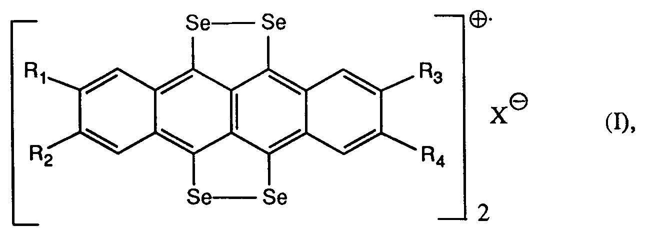
worin R₁, R₂, R₃ und R₄ unabhängig voneinander H oder F bedeuten; R₁ für CH₃ und R₂, R₃ und R₄ für H stehen; R₁, R₂, R₃ und R₄ je für CH₃ stehen; oder R₁ und R₂ CH₃ oder Cl und R₃ und R₄ H darstellen, und X für Cl oder Br steht, das dadurch gekennzeichnet ist, dass man ein Tetraselenotetracen der Formel II 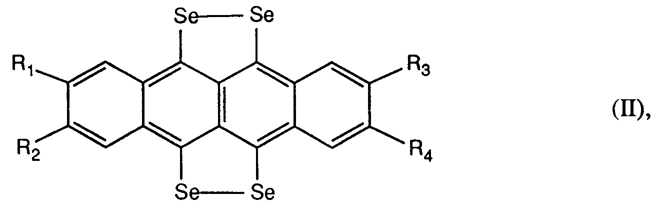
in einem polaren aprotischen Lösungsmittel mit stöchiometrischen Mengen eines Ammoniumhydrochlorids oder -bromids in Gegenwart von Sauerstoff bei erhöhter Temperatur umsetzt.

In Formel I bedeutet X bevorzugt Cl.

Bevorzugt verwendete Verbindungen der Formel II sind 2-Fluortetraselenotetracen, 2,3-Difluortetraselenotetracen, 2,3,8,9-Tetrafluor- oder 2,3,8,9-Tetramethyltetraselenotetracen, und besonders Tetraselenotetracen.

Bevorzugte inerte sind Lösungsmittel N-alkylierte Säureamide und Lactame. Einige Beispiele für solche Lösungsmittel sind zum Beispiel Tetramethylharnstoff, Hexamethylphosphorsäuretriamid, Dimethylformamid, Dimethylacetamid und N-Methylpyrrolidon. Besonders bevorzugt wird N-Methylpyrrolidon verwendet. Die verwendeten Lösungsmittel enthalten vorteilhaft geringe Mengen, zum Beispiel 0,1 bis 10 Gew.-%, bevorzugter 0,5 bis 5 Gew.-% Wasser zur Verbesserung der Löslichkeit der verwendeten Ammonium-Hydrohalogenide.

Stöchiometrische Mengen bedeutet, dass man auf 1 Moläquivalent eines Tetraselenotetracens mindestens 1/2 Moläquivalent eines Ammonium-Hydrochlorids oder -bromids und mindestens 1/2 Moläquivalent Sauerstoff verwendet. Man kann auch einen Überschuss, zum Beispiel einen bis zu zehnfachen Überschuss des Ammonium-Hydrochlorids oder -bromids verwenden.

Der Sauerstoff kann in reiner Form, als Luft oder im Gemisch mit einem Inertgas wie zum Beispiel Stickstoff, Kohlendioxid, Helium, Neon oder Argon durch die Reaktionslösung geleitet werden. In einer anderen Ausführungsform kann man dem Reaktionsgemisch Sauerstoff abgegebende Oxidationsmittel zugeben, zum Beispiel Metallperoxide, Wasserstoffperoxid oder anorganische Peroxosalze. Einige Beispiele für solche Oxidationsmittel sind Alkalimetallperoxide wie Natriumperoxid, und Alkalimetall- oder Ammoniumdisulfat, zum Beispiel Na₂S₂O₈ oder (NH₄)₂S₂O₈. Es kann vorteilhaft sein, Sauerstoff abgegebende Oxidationsmittel in Kombination mit reinem gasförmigem Sauerstoff oder Mischungen von Sauerstoff mit einem Inertgas zu verwenden. Bevorzugt verwendet man reinen Sauerstoff, Luft oder H₂O₂ oder Sauerstoff beziehungsweise Luft in Kombination mit H₂O₂.

In einer bevorzugten Ausführungsform wird dem Reaktionsgemisch zur Beschleunigung der Reaktion zusätzlich Wasserstoffperoxid zugesetzt, zum Beispiel in einer Menge von mindestens 20 % der Menge des Ammonium-Hydrochlorids oder -Hydrobromids oder bis zu einem zehnfachen Überschuss. Im allgemeinen verwendet man 25 % der Menge des Ammonium-Hydrochlorids oder -Hydrobromids oder bis zu einem zehnfachen Überschuss.

Die Reaktion wird bevorzugt bei einer Temperatur von 40 bis 250 °C, besonders bevorzugt bei 70 bis 200 °C und besonders bevorzugt bei 80 bis 150 °C durchgeführt.

Geeignete Ammonium-Hydrochloride sind zum Beispiel Pyridinium-Hydrochlorid und solche der Formel R₅R₆R₇N·HCl, worin R₅ lineares oder verzweigtes C₁-C₈-Alkyl oder C₄-C₈-Cycloalkyl bedeutet und R₆ und R₇ unabhängig voneinander für H stehen oder die Bedeutung von R₅ haben, oder R₅ und R₆ zusammen Tetramethylen, Pentamethylen oder 3-Oxa-1,5-Pentylen darstellen und R₇ H ist oder die Bedeutung von R₅ hat. Das Alkyl enthält bevorzugt 1 bis 4, besonders bevorzugt 1 oder 2 C-Atome. Besonders bevorzugt sind wegen der Flüchtigkeit der entstehenden Amine Hydrochloride der Formel R₅R₆R₇N·HCl, worin R₅, R₆ und R₇ C₁-C₄-Alkyl und besonders Methyl oder Ethyl bedeuten. Ebenso geeignet sind die diesen Ammonium-Hydrochloriden entsprechenden Ammonium-Hydrobromide.

Das erfindungsgemässe Verfahren kann beispielsweise so durchgeführt werden, dass man ein Tetraselenotetracen unter Erwärmen in einem Lösungsmittel löst, wobei man zweckmässig unter einer Schutzgsatmosphäre arbeitet (zum Beispiel Stickstoff, Helium, Neon oder Argon), dann eine Lösung des Ammonium-Halogenids im gleichen Lösungsmittel, das etwas Wasser enthält, zugibt und gegebenenfalls gleichzeitig oder getrennt eine wässrige Wasserstoffperoxidlösung zufügt, und danach Sauerstoff durch das Reaktionsgemisch durchleitet und bei erhöhter Temperatur reagieren lässt. Die Tetraselenotetracen-Halogenide fallen als schwarze feine Kristallnadeln aus, die mit üblichen Verfahren wie Auswaschen und Trocknen gereinigt werden können. Die Tetraselenotetracen-Halogenide können als elektrische Leiter verwendet werden und zum Beispiel als antistatische Mittel Kunststoffen einverleibt werden.

In einer besonders vorteilhaften Ausgestaltungsform des erfindungsgemässen Verfahrens löst man in der Reaktionslösung ein organisches Bindemittel, beschichtet mit dieser Lösung ein Substrat, und verdampft dann das Lösungsmittel bei erhöhter Temperatur in Gegenwart von Sauerstoff (reiner Sauerstoff oder Gemischen von Sauerstoff und einem Inertgas, einschliesslich Luft), wobei eine Schicht auf dem Substrat verbleibt, die ein Nadelnetzwerk von Kristallnadeln aus den Verbindungen der Formel I in einer Matrix des organischen Bindemittels enthält. Bei dieser Verfahrensvariante wird zweckmässig Wasserstoffperoxid mitverwendet. Ferner ist es vorteilhaft, Ammonium-Hydrohalogenide von flüchtigen Aminen zu verwenden, so dass man Beschichtungen erhält, die praktisch frei von Reaktionsnebenprodukten sind. Die Herstellung der Reaktionslösung und der Beschichtung wird zweckmässig unter Inertgasatmosphäre vorgenommen, um eine vorzeitige Bildung der Tetraselenotetracen-Halogenide zu verhindern. Freitragende Filme können durch Ablösen von der Unterlage erhalten oder mittels bekannter Giessverfahren direkt und kontinuierlich hergestellt werden. Im Einzelnen kann man bei dieser erfindungsgemässen Variante so vorgehen, dass man das Polymer zusammen mit einem Tetracen der Formel II in einem inerten Lösungsmittel löst und dann mit den übrigen Reaktanden vermischt und darauf ein Substrat beschichtet.

Dieses Verfahren bietet gegenüber dem in der US-A-5 009 812 beschriebenen Verfahren zur Herstellung von elektrisch leitenden Schichten, Filmen oder Folien mit einem Nadelnetzwerk von Tetraselenotetracen-Halogeniden in einer Kunststoffmatrix noch weitere als die zuvor beschriebenen Vorteile. Man erhält sehr homogene Schichten mit guten Leitfähigkeiten in allen Richtungen, weil die Bildung der Halogenide und deren Kristallisation praktisch erst nach dem in einer inerten Atmosphäre durchgeführten Beschichten bei Zufuhr von Sauerstoff einsetzt und damit die Ausrichtung von Kristallnadeln beim Beschichten vermieden werden kann. Ferner erhält man mit diesem Verfahren sehr feinmaschige Nadelnetzwerke, die mechanisch stabiler sind, so dass Filme und Folien zum Beispiel ohne Verlust der elektrischen Leitfähigkeit gerollt werden können. Bei den Beschichtungsverfahren werden auch weder in der Mischanlage noch im Giesskopf oder einer Spraydüse die Ablagerung von Kristallen beobachtet, so dass Unterbrüche der Produktion weitgehend vermieden werden können.

Die Mengen an Tetraselenotetracenen und Bindemitteln in der Reaktionslösung wird zweckmässig so gewählt, dass nach dem Verdampfen des Lösungsmittels die Beschichtung 0,01 bis 10 Gew.-%, bevorzugt 0,3 bis 5 Gew.-% Tetraselenotetracen-Halogenide der Formel I und 99,9 bis 90 Gew.-%, bevorzugt 99,7 bis 95 Gew.-% Bindemittel enthält.

Bei den Bindemitteln handelt es sich bevorzugt um natürliche oder synthetische Kunststoffe. Geeignete Kunststoffe sind zum Beispiel duroplastische, thermoplastische oder strukturell vernetzte Polymere. Bei den thermoplastischen Polymeren kann es sich zum Beispiel um die folgenden Polymeren, Copolymeren bzw. Mischungen von diesen Polymeren handeln:
1. Polymere von Mono- und Diolefinen, beispielsweise Polypropylen, Polyisobutylen, Polybuten-1, Poly-4-methylpenten- 1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylimonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere, und alternierend und statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze).
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylylendiamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamid-systeme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
22. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PCPBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.
23. Polyether aus Diglycidylverbindungen, zum Beispiel Diglycidylethern und Diolen, z. B. aus Bisphenol-A-Diglycidylether und Bisphenol-A.

Bevorzugte Thermoplaste sind Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol.

Bei den Duroplasten und strukturell vernetzten Polymeren kann es sich zum Beispiel um folgende Polymere handeln:
1. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
2. Trocknende und nicht-trocknende Alkydharze.
3. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
4. Vernetzbare Acrylharze, die sich von substituierten Acrylsäureestern ableiten, wie z.B. von Epoxyacrylaten, Urethan-acrylaten oder Polyester-acrylaten.
5. Alkydharze, Polyesterharze und Acrylatharze, die mit Melaminharzen, Harnstoffharzen, Polyisocyanaten oder Epoxidharzen vernetzt sind.
6. Kautschuk auf der Basis von vernetzten Polydienen, zum Beispiel Butadien oder Isopren; Silikonkautschuk.
7. Epoxidharze, die sich von Polyepoxiden ableiten, z.B. von Bisglycidylethern von Polyolen oder von cycloaliphatischen Diepoxiden, und die gegebenenfalls einen Härter als Vernetzungsmittel enthalten, oder die unter Verwendung von Härtungsbeschleunigern thermisch oder durch Einwirkung von Strahlung vernetzt sind.

Unter den vernetzten Polymeren sind vernetzte Epoxidharze bevorzugt, denen als Polyepoxide bevorzugt Glycidylverbindungen mit durchschnittlich zwei Epoxidgruppen im Molekül zu Grunde liegen. Als Glycidylverbindungen kommen vor allem solche mit zwei an ein Heteroatom (z.B. Schwefel, vorzugsweise Sauerstoff oder Stickstoff) gebundenen Glycidylgruppen, β-Methylglycidylgruppen oder 2,3-Epoxycyclopentylgruppen in Frage; genannt seien insbesondere Bis-(2,3-epoxycyclopentyl)-ether, Diglycidylether von mehrwertigen aliphatischen Alkoholen, wie 1,4-Butandiol, oder Polyalkylenglykolen, wie Polypropylenglykole; Diglycidylether von cycloaliphatischen Polyolen, wie 2,2-Bis-(4-hydroxycyclohexyl)-propan; Diglycidylether von mehrwertigen Phenolen, wie Resorcin, Bis-(p-hydroxyphenyl)-methan, 2,2-Bis-(p-hydroxyphenyl)-propan (=Diomethan), 2,2-Bis-(4'-hydroxy-3',5'-dibromphenyl)-propan, 1,3-Di-(p-hydroxyphenyl)-ethan; Di-(β-methylglycidyl)-ether der oben angeführten zweiwertigen Alkohole oder zweiwertigen Phenole; Di-glycidylester von Dicarbonsäuren, wie Phthalsäure, Terephthalsäure, Δ₄-Tetrahydrophthalsäure und Hexahydrophthalsäure; N,N-Diglycidylderivate von primären Aminen und Amiden und heterocyclischen, zwei N-Atome enthaltenden Stickstoffbasen, und N,N'-Diglycidylderivate von disekundären Diamiden und Diaminen, wie N,N-Diglycidylanilin, N,N-Diglycidyltoluidin, N,N-Diglycidyl-p-aminophenyl-methyl-ether, N,N'-Dimethyl-N,N'-diglycidyl-bis-(p-aminophenyl)-methan; N',N"-Diglycidyl-N-phenyl-isocyanurat; N,N'-Diglycidylethylenharnstoff; N,N'-Diglycidyl-5,5-dimethyl-hydantoin, N,N'-Diglycidyl-5-isopropyl-hydantoin, N,N-Methylen-bis-(N',N'-diglycidyl-5,5-dimethylhydantoin), 1,3-Bis-(N-glycidyl-5,5-dimethylhydantoin)-2-hydroxypropan; N,N'-Diglycidyl-5,5-dimethyl-6-isopropyl-5,6-dihydro-uracil, Triglycidylisocyanurat.

Eine bevorzugte Gruppe von Epoxidharzen sind glycidylierte Novolake, Hydantoine, Aminophenole, Bisphenole und aromatische Diamine oder cycloaliphatische Epoxidverbindungen. Besonders bevorzugte Epoxidharze sind glycidylierte Kresolnovolake, Bisphenol-A- und Bisphenol-F-diglycidylether, Hydantoin-N,N'-bisglycid, p-Aminophenoltriglycid, Diaminodiphenylmethantetraglycid, Vinylcyclohexendioxid, 3,4-Epoxycyclohexylmethyl-3,4-epoxycyclohexancarboxylat oder Mischungen hiervon.

Geeignet sind auch vorreagierte Addukte solcher Epoxidverbindungen mit Epoxidhärtern, zum Beispiel ein Addukt aus Bisphenol-A-diglycidylether und Bisphenol-A, oder mit Oligoestern mit zwei terminalen Carboxylgruppen und Epoxiden vorreagierte Addukte.

Als Härter für Epoxidharze kommen saure oder basische Verbindungen in Frage. Als geeignete Härter seien zum Beispiel genannt: mehrwertige Phenole (Resorcin, 2,2-Bis-(4-hydroxyphenyl)-propan) oder Phenol-Formaldehyd-Harze; mehrbasische Carbonsäuren und ihre Anhydride, z. B. Phthalsäureanhydrid, Tetrahydrophthalsäureanhydrid, Hexahydrophthalsäureanhydrid, 4-Methylhexahydrophthalsäureanhydrid, 3,6-Endomethylen-tetrahydrophthalsäreanhydrid, 4-Methyl-3,6-endomethylen-tetrahydrophthalsäureanhydrid (Methylnadicanhydrid), 3,4,5,6,7,7-Hexachlor-endomethylen-tetrahydrophthalsäureanhydrid, Bernsteinsäureanhydrid, Adipinsäureanhydrid, Trimethyladipinsäureanhydrid, Sebacinsäureanhydrid, Maleinsäureanhydrid, Dodecylbernsteinsäureanhydrid, Pyromellitsäuredianhydrid, Trimellitsäureanhydrid, Benzophenontetracarbonsäuredianhydrid, oder Gemische solcher Anhydride.

Eine bevorzugte Gruppe von Härtern sind Novolake und Polycarbonsäureanhydride.

Die Epoxidharze können auch zusätzlich mit Härtungsbeschleunigem oder nur mit thermischen Härtungskatalysatoren gehärtet werden. Beispiele sind 3-Ethyl-4-methylimidazol, Triamylammoniumphenolat); Mono- oder Polyphenole (Phenol, Diomenthan, Salicylsäure); Bortrifluorid und seine Komplexe mit organischen Verbindungen wie z. B. Bortrifluorid-Etherkomplexe und BortrifluoridAmin-Komplexe (BF₃-Monoethylamin-Komplex); Phosphorsäure und Triphenylphosphit.

Härtungsbeschleuniger und Katalysatoren werden üblicherweise in einer Menge von 0,1 bis 10 Gew.-% zugegeben, bezogen auf das Epoxidharz. Härter für Epoxidharze werden im allgemeinen in äquimolaren Mengen verwendet, bezogen auf die Epoxidgruppen und funktionellen Gruppen eines Härters.

Die Kunsstoffe können weitere Additive zur Verbesserung der Verarbeitungseigenschaften, der mechanischen, elektrischen und thermischen Eigenschaften, der Oberflächeneigenschaften und der Lichtstabilität einverleibt sein, zum Beispiel feinteilige Füllstoffe, Verstärkerfüllstoffe, Weichmacher, Gleit- und Entformungsmittel, Haftvermittler, Antistatika, Antioxidantien, Wärme- und Lichtstabilisatoren, Pigmente und Farbstoffe.

Eine bevorzugte Ausführungsform der mit dem erfindungsgemässen Verfahren hergestellten, ein Tetraselenoteracen-Halogenid enthaltenden Kunststoffe (nachfolgend als Zusammensetzung bezeichnet) ist dadurch gekennzeichnet, dass sie als Filme, Folien, Fasern, oder als Beschichtungen auf mindestens einer Oberfläche eines Substrats ausgebildet sind.

In einer besonders bevorzugten Ausführungsform ist die Zusammensetzung als Film oder Folie oder als Beschichtung auf mindestens einer Oberfläche eines Substrats ausgebildet. Zur Herstellung solcher Ausführungsformen wird zum Beispiel ein thermoplastisches Polymer oder mindestens ein Ausgangsprodukt für einen Duroplasten oder ein strukturell vernetztes Polymer in einem inerten Lösungsmittel gelöst oder suspendiert und mit der erfindungsgemäss zu verwendenden Reaktionsmischung versetzt, danach mittels bekannter Beschichtungstechniken auf ein gegebenenfalls vorerwärmtes Substrat aufgetragen und darauf unter Erwärmen und Sauerstoffzufuhr das Lösungsmittel entfernt, wobei vernetzbare Mischungen dann noch ausgehärtet werden können. Freitragende Filme und Folien werden durch Ablösen vom Substrat oder mittels Giessverfahren, zum Beispiel Vorhanggiessverfahren erhalten.

Geeignete Substrate sind zum Beispiel Glas, Metalle, Kunststoffe, mineralische und keramische Werkstoffe, Holz und Papier. Die Substrate können beliebige äussere Formen aufweisen; es kann sich zum Beispiel um Formkörper, Fäden, Fasern, Gewebe, Stangen, Rohre, Bänder, Folien, Platten, Walzen oder Gehäuse handeln.

Geeignete Beschichtungsverfahren sind zum Beispiel Streichen, Walzen, Rakeln, Giessen, Schleudergiessen, Vorhanggiessen und Sprayen. Besonders bevorzugte Verfahren sind Rakel-, Giess und Sprayverfahren.

Geeignete inerte Lösungsmittel für Polymere beziehungsweise Ausgangsprodukte für Polymere sind zum Beispiel polare und bevorzugt aprotische Lösungsmittel, die alleine oder in Mischungen aus mindestens zwei Lösungsmitteln verwendet werden können. Beispiele sind: Ether (Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol) Nitrile (Acetonitril, Propionitril), und Ketone (Aceton, Methyl-butyl-keton).

Für die Durchführung des erfindungsgemässen Verfahrens in Gegenwart von Kunststoffen oder Ausgangsstoffen von Duroplasten oder strukturell vernetzten Polymeren kann es zweckmässig sein, Mischungen von Lösungsmitteln zu verwenden, besonders Mischungen der obigen Lösungsmittel mit N-alkylierten Säureamiden und Lactamen.

Die Beschichtungsverfahren können zum Beispiel so durchgeführt werden, dass man die einzelnen Bestandteile getrennt löst und erst vor dem gewählten Beschichtungsverfahren vereinigt. Man kann aber auch die Bestandteile in zwei Lösungen, zum Beispiel Lösungsmittel, Polymer und Tetraselenotetracen oder Ammonium-Hydrohalogenid sowie Lösungsmittel, Ammonium-Hydrohalogenid beziehungsweise Tetraselenotetracen herstellen, oder alle Bestandteile in einer Lösung vereinigen.

Die Lösungen werden zweckimässig erwärmt, zum Beispiel auf 40 bis 250 °C. Es ist vorteilhaft, auch das Substrat zu erwärmen, um die Entfernung des Lösungsmittels zu beschleunigen, die im allgemeinen bei Temperaturen von 50 bis 250 °C, bevorzugter 70 bis 200 °C und besonders bevorzugt 80 bis 150 °C vorgenommen wird, bis die Beschichtung trocken ist. Sofern die Beschichtungen zu freitragenden Filmen oder Folien abgelöst werden sollen, kann das Substrat vor der Beschichtung mit Antihaftmitteln behandelt werden.

Es ist auch möglich, reine Schichten von Netzwerken aus Kristallnadeln der CT-Komplexe auf einem Substrat herzustellen, indem man zum Beispiel Reaktionslösungen auf einem Substrat aufbringt und danach das Lösungsmittel verdampft. Solche Schichten können zur Erhöhung der elektrischen Leitfähigkeit elektrochemisch metallisiert werden, zum Beispiel mit Cu, Pt oder Pd. Es kann zweckmässig sein, solche reine Schichten mit einer Schutzschicht aus einem Polymer zu beschichten.

Die Schichtdicken können in einem breiten Rahmen variien, je nach Wahl des Beschichtungsverfahrens. Mit Sprayverfahren können sehr dünne Schichten erhalten werden, während man mit Streich- und Giessverfahren auch dicke Schichten erzielen kann. Die Schichtdicken können zum Beispiel 0,01 bis 5000 µm, bevorzugt 0,1 bis 1000 µm und besonders bevorzugt 0,1 bis 500 µm betragen.

Die Zusammensetzung ist je nach Wahl des Polymers opak oder transparent und sie weist hervorragende elektrische Eigenschaften auf. So weisen die Beschichtungen und Formkörper überraschend eine ausgezeichnete Entladungsfähigkeit auf, die für heterogene Materialien sonst schwierig oder gar nicht zu erreichen ist. Die Zusammensetzung eignet sich daher besonders zur Verwendung als antistatisch ausgerüstete Formteile für die elektrostatische Abschirmung von Bauteilen oder zur Verwendung als antistatisch ausgerüstete Formkörper. Die hohen elektrischen Leitfähigkeiten ermöglichen ferner die Verwendung als elektrische Leiter, zum Beispiel als Elektroden für Displayelemente oder eletronische Bauteile, sowie als Ladungsträger in Kondensatoren. Die Zusammensetzungen weisen auch hervorragende mechanische Festigkeiten und mechanische Gebrauchseigenschaften auf.

Die nachfolgenden Beispiele erläutern die Erfindung näher. Prozente sind Gew.-%, wenn nicht anders angegeben.

### A) Herstellungsbeispiele

### Beispiel A1: Herstellung von (Tetraselenotetracen)₂Cl.

200 mg (0,3704 mMol) Tetraselenotetracen werden bei 140 °C in einem Sulfierkolben unter Argonfluss und Rühren in 120 ml N-Methylpyrrolidon (NMP) gelöst. Dann wird auf 95 °C abgekühlt und 1,77 ml einer NMP-Lösung enthaltend 1 % Trimethylammonium-Hydrochlorid (0,1852 mMol) und 2 % Wasser sowie 0,105 ml dreiprozentiges wässriges Wasserstoffperoxid (0,0926 mMol) zugegeben. Man ersetzt danach den Argonstrom durch einen Sauerstoffstrom und rührt das Reaktionsgemisch für 1,5 Stunden bei 100 °C. Die Titelverbindung fällt in Form feiner schwarzer Kristallnadeln aus. Nach dem Abkühlen auf Raumtemperatur wird der schwarze Niederschlag abfiltriert, mit NMP und Aceton gewaschen und dann bei 50 °C im Hochvakuum getrocknet. Man erhält 152,5 mg (75%) (Tetraselenotetracen)₂Cl, das gemessen am Kristallpulver einen spezifischen Widerstandσ = 24 Ω⁻¹·cm⁻¹ aufweist.

### Beispiel A2: Herstellung von (Tetraselenotetracen)₂Cl.

9,80 g Tetraselenotetracen werden in 6000 g N-Methylpyrrolidon bei 144 °C gelöst. Die Lösung wird kontinuierlich mit 72 ml/min aus dem Vorratsbehälter in eine Mischkammer gefördert und dort mit 1,67 ml/min einer N-Methylpyrrolidon-Lösung, die 0,7% Trimethylamin-Hydrochlorid und 2% Wasserstoffperoxid (30%ig) enthält, vermischt. Das Reaktionsgemisch wird auf Raumtemperatur abgekühlt. Die entstandenen (Tetraselenotetracen)₂chlorid-Kristalle werden abfiltriert und bei 100 °C und 0,1 mbar getrocknet. Die Ausbeute beträgt 7,8 g. Durch eine Röntgenpulveranalyse wird nachgewiesen, dass es sich bei den isolierten Kristallen um (Tetraselenotetracen)₂chlorid handelt.

### Beispiel A3: Herstellung von (2-Fluortetraselenotetracen)₂Cl.

66,8 mg 2-Fluortetraselenotetracen werden bei 140 °C gelöst. Nach 30 Minuten wird die Badtemperatur innehalb von 10 Minuten auf 100 °C gesenkt. Dann wird 600 µl einer N-Methylpyrrolidon-Lösung enthaltend 1 % Trimethylammonium-Hydrochlorid und 2% Wasser zugegeben und anschliessend mit 120 µl 3-%iger Wasserstoffperoxid-Lösung in N-Methylpyrrolidon vermischt. Während des Abkühlens wird über die Reaktionsmischung Luft geleitet; dabei fallen Kristalle aus. Bei Raumtemperatur werden die Nadekristalle abfiltriert und dann 4 Stunden bei 100 °C und 0,1 mbar getrocknet. Es werden 54,4 mg Nadelkristalle mit einer Länge von 50 - 100 µm erhalten. Diese Kristalle sind elektrisch leitend. Mittels Röntgenpulverdiagramm wurden die isolierte Verbindung als (2-Fluortetraselenotetracen)₂Cl identifiziert.

### B) Anwendungsbeispiele

### Beispiel B1: Herstellung eines elektrisch leitenden Polycarbonatfilms.

30 mg Tetraselenotetracen und 2,5 g Polycarbonat werden bei 150 °C in 14,5 g NMP gelöst. Nach etwa 30 Minuten werden 300 µl einer NMP-Lösung enthaltend 1 % Trimethylammonium-Hydrochlorid und 1 % Wasser zugegeben und anschliessend mit 3 µl 30-prozentigem wässrigen Wasserstoffperoxid vermischt. Das heisse Gemisch wird auf eine Glasplatte gerakelt (Nassfilmdicke etwa 270 µm) und das Lösungsmittel bei 110 °C verdampft. Man erhält einen transparenten Polycarbonatfilm, der ein dichtes Nadelnetzwerk von Kristallnadeln aus (Tetraselenotetracen)₂Cl enthält und eine Schichtdicke von etwa 24 µm aufweist. Die Leitfähigkeit beträgt 0, 15 S/cm.

### Beispiel B2: Herstellung eines elektrisch leitenden Polycarbonatfilms.

30 mg Tetraselenotetracen und 2,5 g Polycarbonat werden bei 150 °C in 14,5 g NMP gelöst. Nach etwa 30 Minuten werden 300 µl einer NMP-Lösung enthaltend 1 % Trimethylammonium-Hydrochlorid, 1 % Wasser, 1 % H₂O₂ und 5 % Polycarbonat zugegeben und vermischt. Das heisse Gemisch wird auf eine Glasplatte gerakelt (Nassfilmdicke etwa 270 µm) und das Lösungsmittel bei 110 °C verdampft. Man erhält einen transparenten Polycarbonatfilm, der ein dichtes Nadelnetzwerk von Kristallnadeln aus (Tetraselenotetracen)₂Cl enthält und eine Schichtdicke von etwa 22 µm aufweist. Die Leitfähigkeit beträgt 0,08 S/cm.

### Beispiel B3: Herstellung eines elektrisch leitenden Polycarbonatfilms.

18,1 mg Tetraselenotetracen und 0,9 g Polycarbonat werden bei 150 °C in 24 g NMP gelöst. Nach etwa einer Stunde werden 180 µl einer NMP-Lösung enthaltend 1 % Trimethylammonium-Hydrochlorid und 1 % Wasser, und anschliessend 10 µl einer 3-prozentigen wässrigen H₂O₂-Lösung zugegeben und vermischt. Man sprayt das Gemisch auf eine Glasplatte (Spraybedingungen: Zweistoffdüse aus Stahl, Treibmittel Argon, Spraygeschwindigkeit 4 cm/s, Abstand Düse/Glasplatte etwa 20 cm) und verdampft das Lösungsmittel bei 110 °C. Man erhält einen transparenten Polycarbonatfilm, der ein dichtes Nadelnetzwerk von Kristallnadeln aus (Tetraselenotetracen)₂Cl enthält und eine Schichtdicke von etwa 5 µm aufweist. Die Leitfähigkeit beträgt 0,77 S/cm.

### Beispiel B4: Herstellung eines elektrisch leitenden Polyetherfilms.

44,18 mg Tetraselenotetracen und 3,0 g Polyether (Polyadditionsprodukt von Bisphenol-A-Diglycidylether und Bisphenol-A) werden bei 150 °C in 48 g NMP gelöst. Nach etwa 45 Minuten werden 400 µl einer NMP-Lösung zugegeben, die 1 % Trimethylammonium-Hydrochlorid und 2 % Wasser enthält. Danach werden 300 µl einer 3-prozentigen H₂O₂-Lösung in NMP/Wasser (9:1) zugegeben und die Komponenten durch Rühren vermischt. Man sprayt das Gemisch auf eine Glasplatte (Spraybedingungen: Zweistoffdüse aus Stahl, Treibmittel Argon, Spraygeschwindigkeit 4 cm/s, Abstand Düse/Glasplatte etwa 20 cm) und verdampft das Lösungsmittel bei 100 °C. Man erhält einen transparenten Polyetherfilm, der ein dichtes Nadelnetzwerk von Kristallnadeln aus (Tetraselenotetracen)₂Cl enthält und eine Schichtdicke von etwa 5 µm aufweist. Die Leitfähigkeit beträgt 1 S/cm.

### Beispiel B5: Herstellung eines elektrisch leitenden Polyetherimidfilms.

40 mg Tetraselenotetracen und 4,0 g Polyetherimid werden in 20 g N-Methylpyrrolidon bei 155 °C gelöst. Anschliessend werden 660 µl einer N-Methylpyrrolidon-Lösung enthaltend 0,8 % Trimethylammonium-Hydrobromid, 1,6 % Wasserstoffperoxid (30-%ig) und 2% Wasser zugegeben und vermischt. Das heisse Gemisch wird auf eine vorgeheizte Glasplatte gerakelt (Nassfilmdicke etwa 250 µm) und das Lösungsmittel bei 120 °C verdampft. Man erhält einen transparenten Polyetherimidfilm der ein elektrisch leitendes Netzwerk von Kristallnadeln aus (Tetraselenotetracen)₂-chlorid enthält und eine Schichtdicke von etwa 20 µm aufweist. Die Leitfähigkeit beträgt 0,1 S/cm.

### Beispiel B6: Herstellung eines elektrisch leitenden Polycarbonatfilms.

33,2 mg Tetraselenotetracen und 2,5 g Polycarbonat werden in 17,5 g N-Methylpyrrolidon bei 150 °C gelöst. Nach etwa 50 min werden 450 µl einer N-Methylpyrrolidon-Lösung enthaltend 1 % Trimethylammonium-Hydrobromid und 2 % Wasser zugegeben und anschliessend mit 40 µl 3-%iger Wasserstoffperoxid-Lösung in N-Methylpyrrolidon vermischt. Das heisse Gemisch wird auf eine vorgeheizte Glasplatte gerakelt (Nassfilmdicke etwa 270 µm) und das Lösungsmittel bei 110 °C verdampft. Man erhält einen transparenten Polycarbonatfilm, der ein dichtes, elektrisch leitendes Netzwerk von Kristallnadeln aus (Tetraselenotetracen)₂bromid enthält und eine Schichtdicke von etwa 20 µm aufweist. Die Leitfähigkeit beträgt 0,2 S/cm.

### Beispiel B7: Herstellung eines elektrisch leitenden Polyetherfilms.

13,6 mg Tetraselenotetracen und 1,5 g Polyether (Polyadditionsprodukt von Bisphenol-A-Diglycidylether und Bisphenol-A) werden bei 150 °C in 25 g N-Methylpyrrolidon gelöst. Nach etwa 1 Stunde werden 140 µl einer N-Methylpyrrolidon-Lösung zugegeben, die 1% Trimethylammonium-Hydrochlorid und 1% H₂O enthält, anschliessend 50 µl einer 3%-igen H₂O₂-Lösung in N-Methylpyrrolidon zugegeben und durch Rühren vermischt. Man sprayt das Gemisch auf eine vorgeheizte Glasplatte (Spraybedingungen: Zweistoffdüse aus Stahl, Treibmittel: Argon, Spraygeschwindigkeit: 4 cm/s, Abstand Düse zu Glasplatte etwa 20 cm) und verdampft das Lösungsmittel bei 100 °C. Man erhält je einen transparenten Polyetherfilm, der ein dichtes Netzwerk von Kristallnadeln aus (Tetraselenotetracen)₂chlorid enthält und eine Schichtdicke von etwa 7 µm aufweist.

## Patentansprüche

1. Verfahren zur Herstellung von Tetraselenotetracenchloriden oder -bromiden der Formel I, 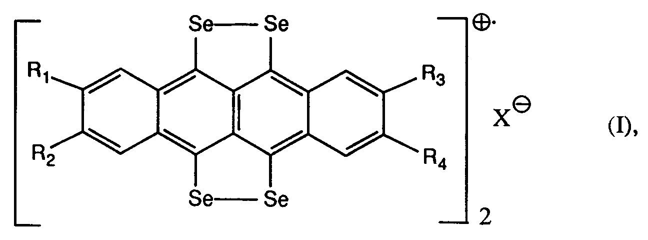 worin R₁, R₂, R₃ und R₄ unabhängig voneinander H oder F bedeuten; R₁ für CH₃ und R₂, R₃ und R₄ für H stehen; R₁, R₂, R₃ und R₄ je für CH₃ stehen; oder R₁ und R₂ CH₃ oder Cl und R₃ und R₄ H darstellen, und X für Cl oder Br steht, das dadurch gekennzeichnet ist, dass man ein Tetraselenotetracen der Formel II 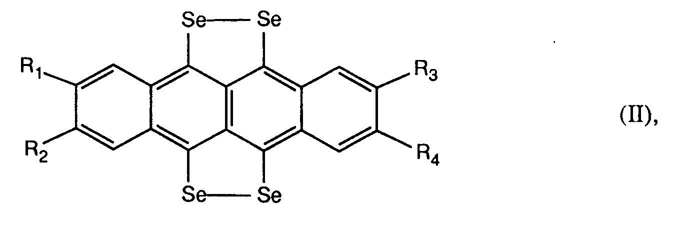 in einem polaren aprotischen Lösungsmittel mit stöchiometrischen Mengen eines Ammoniumhydrochlorids oder -bromids in Gegenwart von Sauerstoff bei erhöhter Temperatur um setzt.

2. Verfahren gemäss Anspruch 1, dadurch gekennzeichnet, dass X in Formel I für Cl steht.

3. Verfahren gemäss Anspruch 1, dadurch gekennzeichnet, dass man 2-Fluortetraselenotetracen, 2,3-Difluortetraselenotetracen, 2,3,8,9-Tetrafluor- oder 2,3,8,9-Tetramethyltetraselenotetracen oder Tetraselenotetracen als Verbindungen der Formel II verwendet.

4. Verfahren gemäss Anspruch 3, dadurch gekennzeichnet, dass man Tetraselenotetracen als Verbindung der Formel II verwendet.

5. Verfahren gemäss Anspruch 1, dadurch gekennzeichnet, dass man N-alkylierte Säureamide und Lactame als Lösungsmittel verwendet.

6. Verfahren gemäss Anspruch 5, dadurch gekennzeichnet, dass man N-Methylpyrrolidon als Lösungsmittel verwendet.

7. Verfahren gemäss Anspruch 1, dadurch gekennzeichnet, dass man auf 1 Moläquivalent eines Tetraselenotetracens mindestens 1/2 Moläquivalent eines Ammonium-Hydrochlorids oder -bromids und mindestens 1/2 Moläquivalent Sauerstoff verwendet.

8. Verfahren gemäss Anspruch 1, dadurch gekennzeichnet, dass man reinen Sauerstoff oder Luft verwendet.

9. Verfahren gemäss Anspruch 1, dadurch gekennzeichnet, dass man dem Reaktionsgemisch Wasserstoffperoxid zusetzt oder die Reaktion nur mit Wasserstoffperoxid durchführt.

10. Verfahren gemäss Anspruch 9, durch gekennzeichnet, dass das Wasserstoffperoxid in einer Menge von mindestens 20 % der Menge des Ammonium-Hydrochlorids oder -Hydrobromids oder bis zu einem zehnfachen Überschuss verwendet wird.

11. Verfahren gemäss Anspruch 1, dadurch gekennzeichnet, dass man die Reaktion bei einer Temperatur von 40 bis 250 °C durchführt.

12. Verfahren gemäss Anspruch 11, dadurch gekennzeichnet, dass man die Reaktion bei einer Temperatur von 70 bis 200 °C durchführt.

13. Verfahren gemäss Anspruch 11, dadurch gekennzeichnet, dass man die Reaktion bei einer Temperatur von 80 bis 150 °C durchführt.

14. Verfahren gemäss Anspruch 1, dadurch gekennzeichnet, dass man als Ammonium-Hydrochloride und Ammonium-Hydrobromide Pyridinium-Hydrochlorid oder solche der Formel R₅R₆R₇N·HCl, worin R₅ lineares oder verzweigtes C₁-C₈-Alkyl oder C₄-C₈-Cy-cloalkyl bedeutet und R₆ und R₇ unabhängig voneinander für H stehen oder die Bedeutung von R₅ haben, oder R₅ und R₆ zusammen Tetramethylen, Pentamethylen oder 3-Oxa-1,5-Pentylen darstellen und R₇ H ist oder die Bedeutung von R₅ hat, oder die entsprechenden Hydrobromide verwendet.

15. Verfahren gemäss Anspruch 14, dadurch gekennzeichnet, dass das Alkyl 1 bis 4 C-Atome enthält.

16. Verfahren gemäss Anspruch 14, dadurch gekennzeichnet, dass die Hydrochloride der Formel R₅R₆R₇N·HCl entsprechen, worin R₅, R₆ und R₇ C₁-C₄-Alkyl bedeuten.

17. Verfahren gemäss Anspruch 14, dadurch gekennzeichnet, dass R₅, R₆ und R₇ für Methyl oder Ethyl stehen.

18. Verfahren gemäss Anspruch 1, dadurch gekennzeichnet, dass in der Reaktionslösung ein organisches Bindemittel gelöst ist, mit dieser Lösung ein Substrat beschichtet wird, und danach das Lösungsmittel bei erhöhter Temperatur in Gegenwart von Sauerstoff verdampft wird, wobei eine Schicht auf dem Substrat verbleibt, die ein Nadelnetzwerk von Kristallnadeln aus (Tetraselenotetracen)₂Cl oder (Tetraselenotetracen)₂Br in einer Matrix des organischen Bindemittels enthält.

19. Verfahren gemäss Anspruch 18, dadurch gekennzeichnet, dass die Reaktionslösung zusätzlich Wasserstofferoxid enthält.

20. Verfahren gemäss Anspruch 18, dadurch gekennzeichnet, dass die Menge an Tetraselenotetracen und Bindemittel in der Reaktionslösung so gross ist, dass nach dem Verdampfen des Lösungsmittels die Beschichtung 0,01 bis 10 Gew.-% Tetraselenotetracen-Halogenide der Formel I und 99,9 bis 90 Gew.-% Bindemittel enthält.

21. Verfahren gemäss Anspruch 18, dadurch gekennzeichnet, dass die Herstellung der Reaktionslösung und der Beschichtung unter Inertgasatmosphäre vorgenommen wird.

22. Verfahren gemäss Anspruch 18, dadurch gekennzeichnet, dass es sich bei dem Bindemittel um ein thermoplastisches Polymer oder um Ausgangsstoffe für duroplastische oder strukturell vernetzte Polymere handelt, die nach der Beschichtung gehärtet oder vernetzt werden.

23. Verfahren gemäss Anspruch 22, dadurch gekennzeichnet, dass es sich bei dem thermoplastischen Polymer um Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol handelt.

24. Verfahren gemäss Anspruch 18, dadurch gekennzeichnet, dass die Schicht zur Herstellung eines Films oder einer Folie vom Substrat abgelöst wird.

25. Verfahren gemäss Anspruch 24, dadurch gekennzeichnet, dass es als Giessverfahren kontinuierlich durchgeführt wird.
